(19) Europäisches Patentamt
European Patent Office
Office européen des brevets

(11) Veröffentlichungsnummer: **0 537 589 A2**

(12) # EUROPÄISCHE PATENTANMELDUNG

(21) Anmeldenummer: **92117002.3**

(22) Anmeldetag: **06.10.92**

(51) Int. Cl.[5]: **C07C 217/90**, C07C 213/06, C07C 213/02, C07C 205/22

(30) Priorität: **16.10.91 DE 4134147**

(43) Veröffentlichungstag der Anmeldung: **21.04.93 Patentblatt 93/16**

(84) Benannte Vertragsstaaten: **BE CH DE FR GB LI NL**

(71) Anmelder: **BASF Aktiengesellschaft**
**Carl-Bosch-Strasse 38**
**W-6700 Ludwigshafen(DE)**

(72) Erfinder: **Hupfeld, Bernd, Dr.**
**Mausbergweg 6**
**W-6720 Speyer(DE)**
Erfinder: **Aumueller, Alexander, Dr.**
**Rieslingweg 25**
**W-6730 Neustadt(DE)**

(54) **Verfahren zur Herstellung von Dinitro- und Diaminophenoxyverbindungen.**

(57) Verfahren zur Herstellung von Dinitro- und Diaminophenoxyverbindunger der allgemeinen Formel I

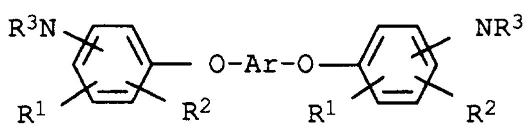

(I),

in der

| | |
|---|---|
| $R^1$, $R^2$ | Wasserstoff, $C_1$- bis $C_4$-Alkyl und $C_1$- bis $C_4$-Alkoxy, |
| $R^3$ | $H_2$ oder $O_2$ und |
| Ar | Phenyl, Naphthyl, Biphenyl oder einen der Reste |

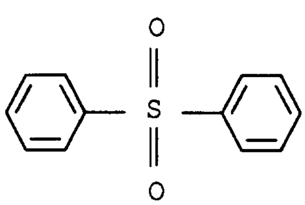
,
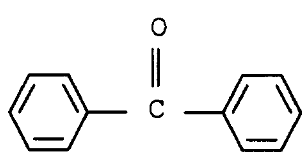
,
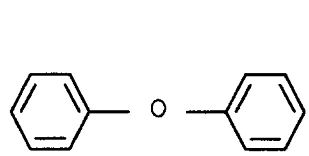
,

oder 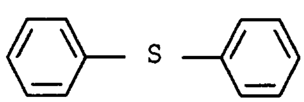

bedeuten, durch Umsetzung von aromatischen Dihydroxyverbindungen der allgemeinen Formel II

HO-Ar-OH (II),

worin Ar die oben angegebene Bedeutung hat, mit einem Nitroaromaten der allgemeinen Formel III

EP 0 537 589 A2

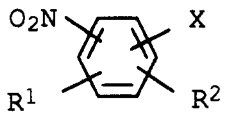

(III),

in der $R^1$ und $R^2$ die obengenannten Bedeutungen haben und X für Halogen steht, in einem Lösungsmittel in Gegenwart einer Base bei Temperaturen von 120 bis 200 °C, dadurch gekennzeichnet, daß man als Lösungsmittel N-Methylpyrrolidon verwendet und die so erhaltenen Nitroverbindungen gegebenenfalls nach bekannten Verfahren zu den entsprechenden Aminen reduziert.

Die Erfindung betrifft ein verbessertes Verfahren zur Herstellung von Dinitro- und Diaminophenoxyverbindungen durch Umsetzung von aromatischen Dihydroxyverbindungen mit Nitroaromaten in N-Methylpyrrolidon als Lösungsmittel in Gegenwart einer Base bei erhöhten Temperaturen und gegebenenfalls Produktion der so erhaltenen Nitroverbindungen nach bekannten Verfahren zu den entsprechenden Aminen.

In der US-A-3 687 663 ist die Herstellung von 2,2-Bis-(4-aminophenoxy-4'-phenyl)-propan beschrieben. Dabei wird 1-Chlor-4-nitrobenzol mit Bisphenol A (2,2-Bis-(4-hydroxyphenyl)-propan) in Dimethylformamid in Gegenwart von Natriumcarbonat umgesetzt und die erhaltene Dinitroverbindung zum Diamin hydriert. Nachteilig bei diesem Verfahren ist die lange Reaktionszeit (18 h) zur Herstellung der Dinitroverbindung, ein 20 mol-%iger Überschuß an eingesetztem 1-Chlor-4-nitrobenzol, der verlorengeht, sowie die niedrige Ausbeute von 84 %.

In Chem. Abstr. Vol. 110, 213-598 ist neben der Herstellung von 2,2-Bis-(4-aminophenoxy-4'-phenyl)-propan auch die Herstellung von 1,3-Bis-(4-aminophenoxy)-benzol und 1,4-Bis-(4-aminophenoxy)-benzol beschrieben. Die Herstellung der Dinitroverbindungen erfolgt wie in der US-A-3 687 663 in Dimethylformamid in Gegenwart von Natriumcarbonat.

In der DE-A-27 26 541 ist die Herstellung von 2,2'-Bis-(4-aminophenoxy)-biphenyl beschrieben. Umgesetzt wird 1-Chlor-4-nitrobenzol mit 2,2'-Dihydroxybiphenyl in Dimethylsulfoxid als Lösungsmittel und Kaliumhydroxid als Base. Die Dinitroverbindung wird in Ausbeuten von lediglich 15 % bzw. 23 % erhalten.

Der vorliegenden Erfindung lag daher die Aufgabe zugrunde, den zuvor genannten Nachteilen abzuhelfen.

Demgemäß wurde ein neues und verbessertes Verfahren zur Herstellung von Dinitro- und Diaminophenoxyverbindungen der allgemeinen Formel I

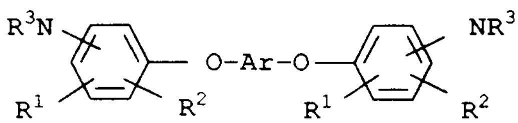

(I),

in der

| | |
|---|---|
| $R^1$, $R^2$ | Wasserstoff, $C_1$- bis $C_4$-Alkyl und $C_1$- bis $C_4$-Alkoxy, |
| $R^3$ | $H_2$ oder $O_2$ und |
| Ar | Phenyl, Naphthyl, Biphenyl oder einen der Reste |

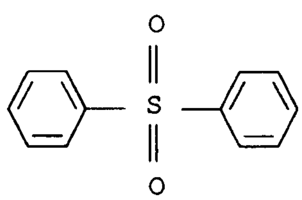 , 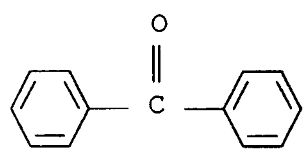 , 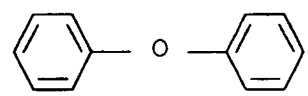 ,

oder 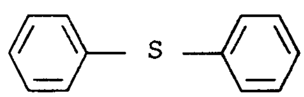

bedeuten, durch Umsetzung von aromatischen Dihydroxyverbindungen der allgemeinen Formel II

HO-Ar-OH (II),

worin Ar die oben angegebene Bedeutung, hat mit einem Nitroaromaten der allgemeinen Formel III

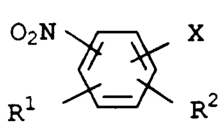

(III),

in der $R^1$ und $R^2$ die obengenannten Bedeutungen haben und X für Halogen steht, in einem Lösungsmittel in Gegenwart einer Base bei Temperaturen von 120 bis 200 ° C gefunden, welches dadurch gekennzeichnet ist, daß man als Lösungsmittel N-Methylpyrrolidon verwendet und die so erhaltenen Nitroverbindungen gegebenenfalls nach bekannten Verfahren zu den entsprechenden Aminen reduziert.

Die Vorteile der vorliegenden Erfindung sind

a) kurze Reaktionszeiten (ca. 2 bis 4 h) bei weitgehend vollständigem Umsatz der Edukte,
b) stöchiometrischer Einsatz der Edukte und
c) annähernd quantitative Ausbeuten der Dinitrophenoxyverbindungen.

Das erfindungsgemäße Verfahren kann wie folgt durchgeführt werden:

Ein Nitroaromat III, eine aromatische Dihydroxyverbindung II und eine Base können bei Raumtemperatur im Lösungsmittel N-Methylpyrrolidon vorgelegt und anschließend unter Rühren auf Temperaturen von 120 bis 200 ° C, bevorzugt 140 bis 180 ° C, besonders bevorzugt 160 bis 180 ° C erwärmt werden. Der Reaktionsfortgang kann durch Dünnschichtchromatographie verfolgt werden. Nach beendeter Reaktion kann wie üblich aufgearbeitet werden, z.B. läßt man das Reaktionsgemisch abkühlen und in Wasser einlaufen. Von ausgefallenem Wertprodukt kann abfiltriert, der Filterrückstand mit Wasser gewaschen und getrocknet werden.

Die Reaktion kann bei Drücken von 0,01 bis 50 bar, vorzugsweise bei 0,5 bis 5 bar, besonders bevorzugt bei Normaldruck (Atmosphärendruck) durchgeführt werden.

Als Basen eignen sich z.B. Alkali- und Erdalkalicarbonate, bevorzugt Alkalicarbonate wie Lithiumcarbonat, Natriumcarbonat, Kaliumcarbonat, Caesiumcarbonat, besonders bevorzugt Natrium- und Kaliumcarbonat, sowie Mischungen aus Natrium- und Kaliumcarbonat.

Die erhaltenen Dinitrophenoxyverbindungen I können ohne Reinigung direkt oder nach einer Reinigung nach bekannten Verfahren zu den entsprechenden Diaminophenoxyverbindungen I umgesetzt werden.

Die Umsetzung der Dinitrophenoxyverbindungen I zu den Diaminophenoxyverbindungen I kann z.B. wie folgt erfolgen:

Eine Dinitroverbindung I kann in einem geeigneten Verdünnungsmittel suspendiert oder gelöst, mit einem Katalysator versetzt und in einem Autoklaven gegebenenfalls bei erhöhter Temperatur mit Wasserstoff bei erhöhtem Druck hydriert werden.

Geeignete Verdünnungsmittel sind zum Beispiel: $C_1$- bis $C_8$-Alkanole, bevorzugt $C_1$- bis $C_4$-Alkanole wie Methanol, Ethanol, Propanol, Isopropanol, Butanol, Isobutanol, Keton wie Aceton, Ester wie Essigester, aromatische Kohlenwasserstoffe wie Toluol, Formamide wie Dimethylformamid oder Dimethylacetamid.

Als Katalysatoren können zum Beispiel Edelmetalle wie Platin, Palladium oder Rhodium auf Aktivkohle, Raney-Kobalt oder Raney-Nickel eingesetzt werden.

Bei einer bevorzugten Ausführungsform wird die Dinitroverbindung I in einem Alkohol als Verdünnungsmittel und Platin oder Palladium auf Aktivkohle als Katalysator umgesetzt. Hydriert wird bei 40 bis 90 ° C bei einem Wasserstoffdruck von 1 bis 80 bar.

Besonders bevorzugt ist die Umsetzung der Dinitroverbindung in Dimethylformamid als Verdünnungsmittel und Raney-Nickel als Katalysator. Hydriert wird bei einer Temperatur von 60 bis 90 ° C und einem Wasserstoffdruck von 60 bis 100 bar.

Die Substituenten $R^1$, $R^2$, $R^3$ und X sowie das Brückenglied Ar haben folgende Bedeutung:

$R^1$, $R^2$ unabhängig voneinander

- Wasserstoff,
- $C_1$- bis $C_4$-Alkyl wie Methyl, Ethyl, n-Propyl, iso-Propyl, n-Butyl, iso-Butyl, sec.-Butyl und tert.-Butyl,
- $C_1$- bis $C_4$-Alkoxy wie Methoxy, Ethoxy, n-Propoxy, iso-Propoxy, n-Butoxy, iso-Butoxy, sec.-Butoxy und tert.-Butoxy,

$R^3$

- $H_2$,
- $O_2$,

Ar

- Phenyl, Naphthyl, Biphenyl oder einen der Reste

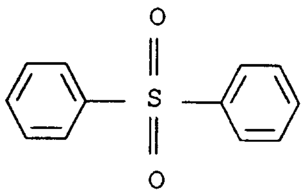
,
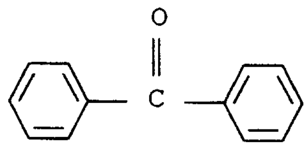
,
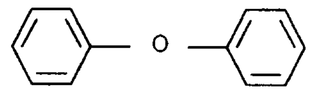

oder
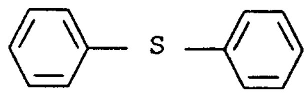

wobei sich die Substituenten in ortho-, meta- oder para-Stellung befinden können.

Beispiele

Allgemeine Arbeitsvorschrift:

In einem 4 l-Kolben mit Rührer werden bei Raumtemperatur unter einer Stickstoffatmosphäre 1,56 Mol Dihydroxyaromat, 3,12 Mol einer Verbindung der allgemeinen Formel III, 3,25 Mol (448,8 g) Kaliumcarbonat und 1,5 l NMP vorgelegt und anschließend auf 160°C-175°C unter Rühren erwärmt. Der Umsatz der Edukte wird durch Dünnschichtchromatographie verfolgt. Nach beendeter Reaktion wird das Reaktionsgemisch auf unter 100°C abgekühlt und in 4,5 l schnell gerührtes Wasser eingetragen. Vom ausgefallenen Wertprodukt wird abgesaugt, der Filterrückstand mit 2 l Wasser gewaschen und im Vakuumschrank getrocknet. Ausbeuten und Reinheiten der dargestellten Dinitroverbindungen sind der folgenden Tabelle zu entnehmen.

Tabelle 1

| Beispiel | Dihydroxyaromat | Verbindung der Formel III | Dinitroverbindung | Reaktionszeit (h) | Ausbeute % | Gehalt % (HPLC) |
|---|---|---|---|---|---|---|
| 1 | 1,3-Dihydroxybenzol | 1-Chlor-4-nitrobenzol | 1,3-Bis-(4-nitrophenoxy)-benzol | 2,5 | 99,2 | 98,9 |
| 2 | 1,4-Dihydroxybenzol | 1-Chlor-4-nitrobenzol | 1,4-Bis-(4-nitrophenoxy)-benzol | 2,5 | 98,9 | 98,7 |
| 3 | 1,2-Dihydroxybenzol | 1-Chlor-4-nitrobenzol | 1,2-Bis-(4-nitrophenoxy)-benzol | 2,5 | 99,5 | 99,2 |
| 4 | 2,2-Bis-(4-hydroxyphenyl)-propan | 1-Chlor-4-nitrobenzol | 2,2-Bis-(4-nitrophenoxy-4'-phenyl)-propan | 3,5 | 98,6 | 98,8 |
| 5 | 4,4'-Dihydroxybiphenyl | 1-Chlor-4-nitrobenzol | 4,4'-Bis-(4-nitrophenoxy)-biphenyl | 3 | 99,1 | 99,4 |
| 6 | 2,2'-Dihydroxybiphenyl | 1-Chlor-4-nitrobenzol | 2,2'-Bis-(4-nitrophenoxy)-biphenyl | 3 | 99,3 | 99,2 |
| 7 | 4,4'-Dihydroxydiphenylsulfid | 1-Chlor-4-nitrobenzol | 4,4'-Bis-(4-nitrophenoxy-4'-phenyl)-sulfid | 3 | 98,8 | 98,9 |
| 8 | 2,7-Dihydroxynaphthalin | 1-Chlor-4-nitrobenzol | 2,7-Bis-(4-nitrophenoxy)-naphthalin | 3,5 | 98,7 | 98,9 |
| 9 | 1,6-Dihydroxynaphthylin | 1-Chlor-4-nitrobenzol | 1,6-Bis-(4-nitrophenoxy)-naphthalin | 3,5 | 98,9 | 98,7 |
| 10 | 2,3-Dihydroxynaphthalin | 1-Chlor-4-nitrobenzol | 2,3-Bis-(4-nitrophenoxy)-naphthalin | 3,5 | 98,6 | 98,6 |
| 11 | 2,2'-Dihydroxybiphenyl | 1-Chlor-2-nitrobenzol | 2,2'-(2-nitrophenoxy)-biphenyl | 4 | 97,8 | 97,6 |

Allgemeine Vorschrift zur Hydrierung der in Tabelle 1 angegebenen Dinitroverbindungen: 500 g Dinitroverbindung werden in 2 l Dimethylformamid in Gegenwart von 25 g Raney-Nickel bei 60 bis 90 °C und einem Wasserstoffdruck von 60 bis 100 bar hydriert. Nach beendeter Hydrierung wird vom Raney-Nickel abfiltriert und die Reaktionsmischung in 6 l schnell gerührtes Wasser eingetragen. Vom ausgefallenen Wertprodukt wird abgesaugt, der Filterrückstand mit 2 l Wasser gewaschen und im Vakuum getrocknet.

Ausbeuten und Reinheiten sind der folgenden Tabelle zu entnehmen.

Tabelle 2

| Dinitroverbindung aus Beispiel | Diamin | Ausbeute (%) | Reinheit % (HPLC) |
|---|---|---|---|
| 1 | 1,3-Bis-(4-aminophenoxy)-benzol | 97,9 | 98,2 |
| 2 | 1,4-Bis-(4-aminophenoxy)-benzol | 98,4 | 98,4 |
| 3 | 1,2-Bis-(4-aminophenoxy)-benzol | 98,6 | 98,5 |
| 4 | 2,2-Bis-(4-aminophenoxy)-4'-phenyl)-propan | 97,8 | 98,5 |
| 5 | 4,4'-Bis-(4-aminophenoxy)-biphenyl | 99,2 | 98,9 |
| 6 | 2,2'-Bis-(4-aminophenoxy)-biphenyl | 99,3 | 99,0 |
| 7 | 4,4'-Bis-(4-aminophenoxy-4'-phenyl)-sulfid | 96,1 | 97,1 |
| 8 | 2,7-Bis-(4-aminophenoxy)-naphthalin | 98,5 | 99,1 |
| 9 | 1,6-Bis-(4-aminophenoxy)-naphthalin | 98,9 | 99,2 |
| 10 | 2,3-Bis-(4-aminophenoxy)-naphthalin | 98,8 | 98,7 |
| 11 | 2,2'-Bis-(2-aminophenoxy)-biphenyl | 97,3 | 97,1 |

**Patentansprüche**

**1.** Verfahren zur Herstellung von Dinitro- und Diaminophenoxyverbindungen der allgemeinen Formel I

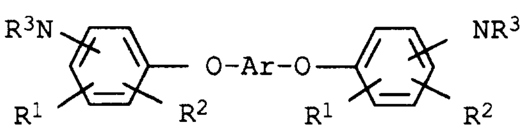

(I),

in der

| | |
|---|---|
| $R^1$, $R^2$ | Wasserstoff, $C_1$- bis $C_4$-Alkyl und $C_1$- bis $C_4$-Alkoxy, |
| $R^3$ | $H_2$ oder $O_2$ und |
| Ar | Phenyl, Naphthyl, Biphenyl oder einen der Reste |

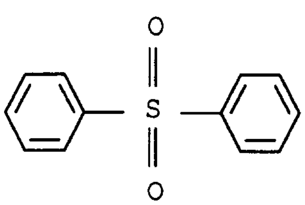
,
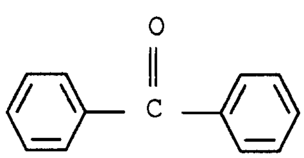
,
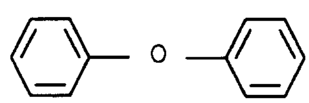
,

oder

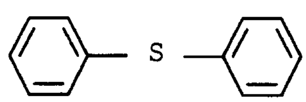

bedeuten, durch Umsetzung von aromatischen Dihydroxyverbindungen der allgemeinen Formel II

HO-Ar-OH (II),

worin Ar die oben angegebene Bedeutung hat, mit einem Nitroaromaten der allgemeinen Formel III

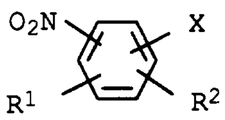

(III),

in der $R^1$ und $R^2$ die obengenannten Bedeutungen haben und X für Halogen steht, in einem Lösungsmittel in Gegenwart einer Base bei Temperaturen von 120 bis 200 ° C, dadurch gekennzeichnet, daß man als Lösungsmittel N-Methylpyrrolidon verwendet und die so erhaltenen Nitroverbindungen gegebenenfalls nach bekannten Verfahren zu den entsprechenden Aminen reduziert.

**2.** Verfahren nach Anspruch 1, dadurch gekennzeichnet, daß als Base Alkalicarbonat verwendet wird.

**3.** Verfahren nach Anspruch 1, dadurch gekennzeichnet, daß man die Umsetzung von II:III:Base im Molverhältnis von 1:2:2 bis 1:2,5:3 durchführt.

**4.** Verfahren nach Anspruch 1, dadurch gekennzeichnet, daß man die Umsetzung bei 140 bis 180 ° C vornimmt.

**5.** Die Verbindung der Formel

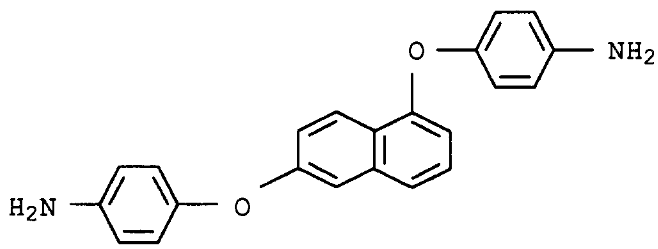

**6.** Die Verbindung der Formel